# DEMANDE DE BREVET EUROPEEN

(11) **EP 0 662 319 A1**
(43) Date de publication de la demande: **12.07.1995**
(21) Numéro de dépôt: 94402933.9
(22) Date de dépôt: 29.12.1994
(51) Int. Cl.: A61K 7/48

(54) **Composition cosmétique et/ou dermatologique pour le traitement du vieillissement contenant des céramides, son utilisation**

(30) Priorité: 10.01.1994 FR 9400173
(71) Demandeur: L'OREAL, F-75008 Paris (FR)
(72) Inventeur: Aubert, Lucien, Les Rocailles, F-06320 Cap d'Ail (FR); Gagnebien-Cabanne, Françoise, F-92320 Chatillon (FR)
(74) Mandataire: Lhoste, Catherine

(57) **Abrégé**

L'invention se rapporte à une utilisation, dans une composition cosmétique ou dermatologique contenant au moins un actif antivieillissement ayant un effet irritant, dans un milieu cosmétiquement ou dermatologiquement acceptable, comme agent apaisant de l'effet irritant, d'un céramide de formule (I) suivante : 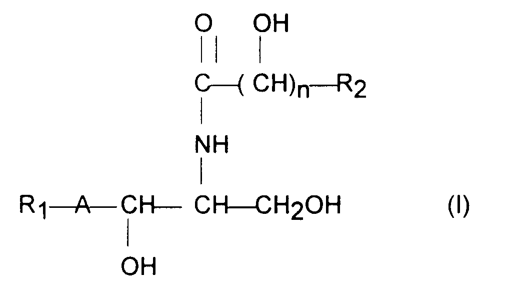
où A représente -CH₂-, 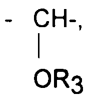
ou -CH=CH- ;
R₁ représente une chaîne alkyle linéaire ou ramifiée, saturée ou insaturée, en C₁₀ à C₂₆ ;
R₂ représente une chaîne alkyle linéaire ou ramifiée, saturée ou insaturée, en C₁₂ à C₃₂ ;
R₃ représente H ou -CO-CHOH-R₂ ;
n représente 0 ou 1.

## Description

L'invention se rapporte à une composition cosmétique et/ou dermatologique pour lutter contre le vieillissement, et en douceur, de la peau du visage et/ou du corps humain, y compris le cuir chevelu. Elle se rapporte aussi à l'utilisation de cette composition pour le traitement des rides et des ridules.

On cherche, de plus en plus, à paraître plus jeune et moins ridé, en utilisant des compositions cosmétiques contenant des actifs capables de lutter contre le vieillissement. Les actifs antivieillissement couramment utilisés sont les α-hydroxy-acides (lactique, glycolique, citrique), les β-hydroxy-acides (acide salicylique, n-octanoyl-5-salicylique) et les rétinoïdes (acide rétinoïque tout trans ou 13-cis, rétinol).

Malheureusement, ces actifs antivieillissement présentent l'inconvénient majeur de provoquer des picotements, des démangeaisons, des tiraillements après leur application, pouvant conduire à un important inconfort. L'utilisation de ces composés pour les utilisateurs à peau sensible est donc souvent rédhibitoire.

Aussi, il subsiste le besoin d'une composition cosmétique et/ou dermatologique pour lutter contre le vieillissement, ne présentant pas ces inconvénients.

L'invention a justement pour objet une utilisation, dans une composition cosmétique ou dermatologique, contenant au moins un actif antivieillissement ayant un effet irritant et un milieu cosmétiquement ou dermatologiquement acceptable, d'un céramide de formule (I) suivante comme agent apaisant de l'effet irritant : 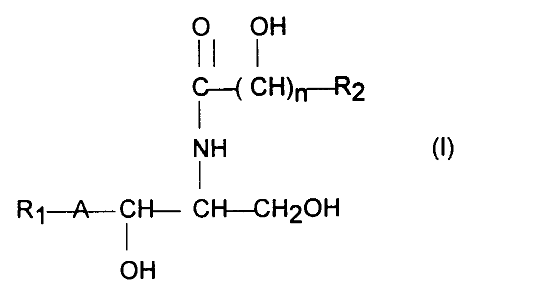
où A représente -CH₂-, 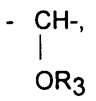
ou -CH=CH- ;
R₁ représente une chaîne alkyle linéaire ou ramifiée, saturée ou insaturée, en C₁₀ à C₂₆ ;
R₂ représente une chaîne alkyle linéaire ou ramifiée, saturée ou insaturée, en C₁₂ à C₃₂ ;
R₃ représente H ou -CO-CHOH-R₂ ;
n représente 0 ou 1.

Ces céramides permettent d'obtenir une composition antivieillissement, douce, applicable par voie topique sur la peau du visage et/ou du corps humain.

Les céramides occupent une place majeure surtout dans les couches supérieures de l'épiderme, c'est-à-dire dans le *stratum corneum*. Il existe plusieurs types de céramides selon leur localisation et leur fonction au sein de l'épiderme. Le terme de céramide, pris dans son sens strict, comprend uniquement les lipides constitués d'une sphingosine reliée à un acide gras ou dérivé d'acide gras par sa fonction amine.

Les céramides dans le *stratum corneum* sont constitués de 6 fractions, chromatographiquement distinctes, ayant une polarité différente selon le degré d'insaturation (qui peut être nul) ou d'hydroxylation de leurs chaînes, leur longueur et leur nombre.

Selon l'invention, on peut utiliser un ou plusieurs céramides de formule (I) éventuellement associés à d'autres types de céramide, comme agents apaisants. De plus, la composition de l'invention peut contenir un ou plusieurs actifs antivieillissement de nature identique ou différente.

Les céramides utilisés notamment dans la composition de l'invention, peuvent être d'origine naturelle ou synthétiques et sont de type II (par exemple la N-oléoyl-dihydrosphingosine), de type III (par exemple la N-stéaroyl phytosphingosine), de type IV (par exemple le N-α-hydroxybéhénoyl-dihydrosphingosine) ou de type V (par exemple la N-α-hydroxypalmitoyl-dihydrosphingosine). On peut aussi utiliser les mélanges de céramides présents dans la peau, décrits dans l'article DOWNING (The Journal of Investigative Dermatology, 84, pp 410-412, 1985).

On peut aussi utiliser comme agent apaisant, une préparation, contenant en plus de ces mélanges de céramides, du cholestérol, des acides gras libres comme l'acide oléique, des triglycérides comme la trioléine et du squalène, afin de retrouver un mélange mimant les lipides épidermiques. Cette préparation peut être utilisée à une concentration allant de 0,01 à 10 % en poids et de préférence de 0,05 à 5 % en poids du total de la composition de l'invention.

A partir de ces céramides simples, on peut, en outre, obtenir des céramides complexes qui peuvent avoir des propriétés similaires à celles des céramides simples. On trouve notamment les sphingolipides tels que les oligoglycocéramides (gangliosides), les monoglycocéramides (cérébrosides), les acylmonoglycocéramides, et les hydroxyacylmonoglycocéramides.

On trouve aussi les sphingophospholipides tels que les sphingomyélines.

Ces céramides simples ou complexes peuvent être d'origine végétale, tels que, par exemple, les glycocéramides de blé vendus par la Société ARD ou un mélange de glycolipides (contenant des glycocéramides, les phospholipides, les triglycérides) vendu sous la dénomination commerciale CERAMIDE VEGETAL de la Société INOCOSM.

La quantité d'agent apaisant utilisée dans l'invention dépend de la quantité d'actif antivieillissement utilisée. Le rapport, en poids, agent apaisant/actif antivieillissement peut par exemple être choisi de 0,0001 à 100000. Par ailleurs, la quantité d'actif représente en pratique de 0,0001% à 20% du poids total de la composition.

L'invention s'applique à tous les actifs antivieillissement présentant un effet irritant. A titre d'exemple d'actifs, auxquels s'applique l'invention peuvent être des α-hydroxy-acides ou des β-hydroxy-acides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxycarbonylés ou alcoxylés ayant de 2 à 18 atomes de carbone.

Comme α-hydroxy-acides utilisables dans l'invention, on peut citer les acides glycolique, lactique, malique, tartrique, citrique, mandélique. Comme β-hydroxy-acides, on peut citer l'acide salicylique ainsi que ses dérivés acylés, les acides hydroxy-2 alcanoïques et leurs dérivés comme l'acide hydroxy-2-méthyl-3-benzoïque et l'acide hydroxy-2-méthoxy-3-benzoïque.

Comme dérivé de l'acide salicylique, on peut citer notamment ceux décrits dans les documents FR-A-2581542 et EP-A-378936, et notamment les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique. On peut aussi utiliser ceux décrits dans le document EP-A-570230.

On peut aussi utiliser comme actifs les α- ou β-céto-acides, les rétinoïdes, l'anthraline, les anthranoïdes (par exemple ceux décrits dans le document EP-A-319028), les peroxydes comme le peroxyde de benzoyle, le minoxidil, la capsaïcine, les sels de lithium et/ou de zinc, les antimétabolites comme le fluoro-5-uracile, les vitamines comme la vitamine C.

Les rétinoïdes auxquels s'applique l'invention sont en particulier le rétinol, les acides rétinoïques tout trans ou 13-cis, le rétinaldéhyde ou les composés cités dans les documents FR-A-2676052, EP-A-210929, EP-A-292348, EP-A-199636, FR-A-2570377, FR-A-2590566, FR-A-2601359, EP-A-325540, EP-A-232199, EP-A-552282, EP-A-284288, EP-A-170105, FR-A-2422677.

Les compositions de l'invention peuvent en outre contenir une huile végétale, minérale (vaseline), siliconée (cyclométhicone), fluorée (perfluoropolyéther) ou synthétique (huile de purcellin), une phase aqueuse, des adjuvants hydrophiles comme les gélifiants (argile, gomme de xanthane), les agents hydratants, cicatrisants comme la glycérine et l'allantoïne ainsi que leurs dérivés et les compositions les contenant, les antioxydants (vitamine E), les conservateurs, les opacifiants, des adjuvants lipophiles tels que des huiles essentielles, des colorants et des parfums, ainsi que des pigments (oxydes de titane ou de zinc) et des charges. On peut aussi trouver des filtres hydrophiles ou lipophiles pour filtrer les rayons visibles et/ou ultraviolets ainsi que des actifs dermatologiques. Ces adjuvants peuvent représenter, au total, de 0,1 % à 10 % du poids total de la composition.

Les compositions selon l'invention peuvent se présenter sous forme d'une solution huileuse, d'un gel aqueux, d'un sérum, d'une lotion, d'une émulsion eau dans huile (E/H) ou huile dans eau (H/E) et/ou d'une dispersion de vésicules lipidiques (ioniques ou non ioniques).

Pour une émulsion, on utilise, selon le cas, un système émulsionnant (E/H) ou (H/E). Lors de l'utilisation d'une dispersion de vésicules lipidiques, ces dernières peuvent constituer le système émulsionnant. La quantité de système émulsionnant est choisie classiquement de 0,1% à 10% du poids total de la composition.

Comme émulsionnant (H/E) utilisable dans l'invention, on peut citer le PEG-50 stéarate et le PEG-40 stéarate vendus respectivement sous les dénominations commerciales MYRJ 53 et MYRJ 52 par la Société ICI et le tristéarate sorbitane vendu sous la dénomination commerciale SPAN 65 par la Société ICI.

Comme émulsionnant (E/H) utilisable dans l'invention, on peut citer le mélange polyglycéryl-4 isostéarate/cétyldiméthicone copolyol/hexyl laurate vendu sous la dénomination commerciale ABIL WE 09 par la Société GOLDSCHMIDT et l'isostéaryl diglycéryl succinate vendu sous la dénomination commerciale IMWITOR 780 K par la Société HÜLS.

L'invention a aussi pour objet une utilisation de la composition définie précédemment pour le traitement des rides et/ou des ridules de la peau, ainsi que pour la préparation d'une pommade destinée au traitement de l'acné, des rides et/ou des ridules de la peau.

D'autres caractéristiques et avantages de l'invention ressortiront mieux des exemples qui suivent et des figures annexées, donnés à titre illustratif et non limitatif. Les proportions sont données en pourcentage pondéral.

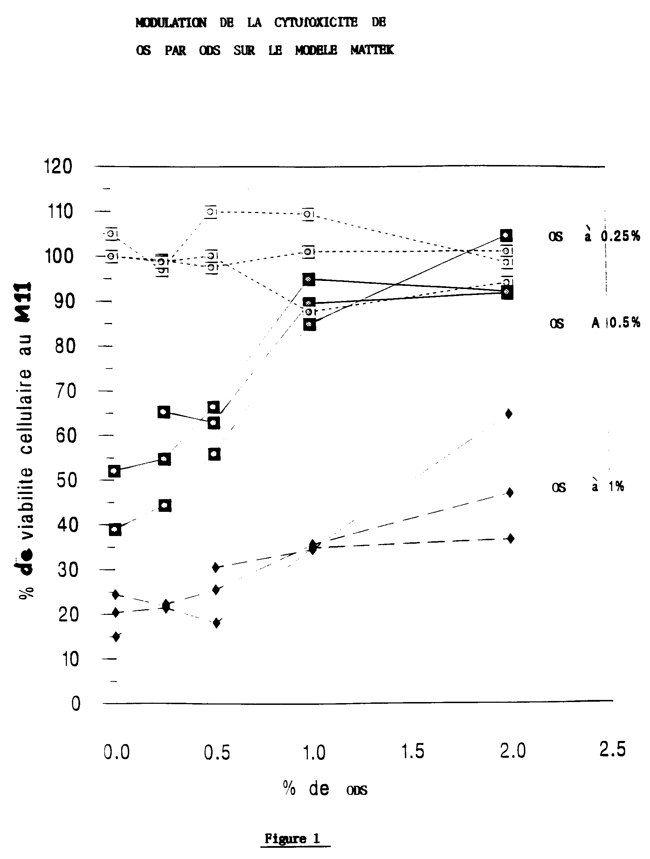

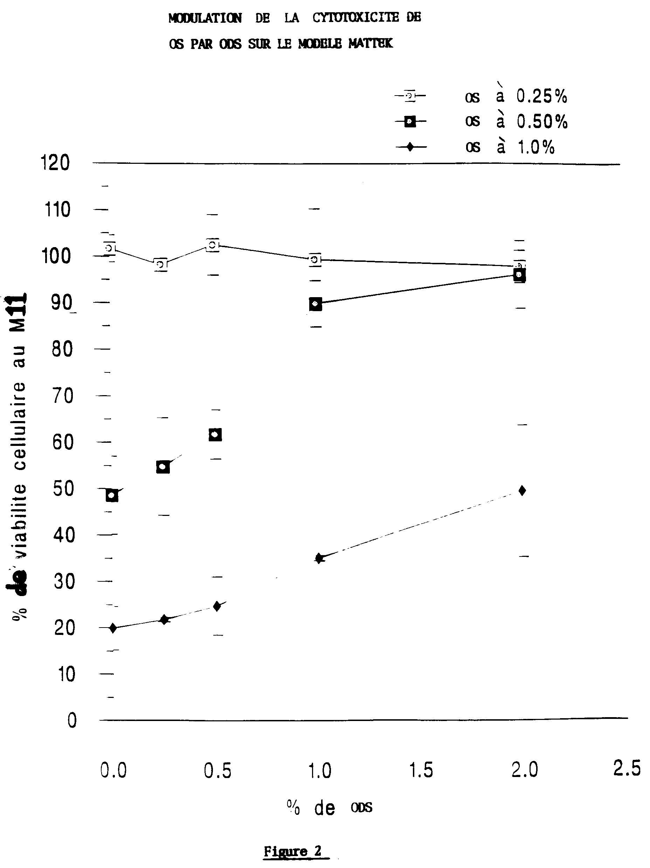

Les figures1 et 2 représentent la modulation de la cytotoxicité de l'acide n-octanoyl-5-salicylique, noté OS, par la N-oléoyl-dihydrosphingosine, noté ODS, sur la peau reconstruite.

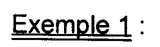

### Phase grasse :

| | |
|---|---|
| - Alcool cétylique | 7 |
| - Glycéryl stéarate | 2,5 |
| - PEG-50 stéarate | 2,5 |
| - Huile d'arachide (apaisant) | 6,2 |
| - Myristate d'isopropyle | 3 |
| - N-oléoyl- dihydrosphingosine (apaisant) | 0,5 |
| - Acide salicylique (actif) | 0,5 |

### Phase aqueuse :

| | |
|---|---|
| - Alcool | 6 |
| - Eau qsp | 100 |

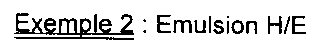

### Phase A :

| | |
|---|---|
| - Acide n-octanoyl-5-salicylique | 1,0 |
| - N-oléoyl-dihydrosphingosine | 0,1 |
| - Huile d'amande douce | 14,5 |
| - Huile de karité | 7,0 |
| - PPG-3 myristyl éther (EMCOL 249-3K) (co-émulsionnant et solvant) | 5,0 |
| - Conservateur (propylparabène) | 0,1 |
| - Polysorbate 60 (TWEEN 60) | 2,5 |
| - Sorbitan stéarate (SPAN 60) | 2,5 |

### Phase B :

| | |
|---|---|
| - Cyclométhicone | 4,0 |
| - Gomme de xanthane | 0,2 |
| - Polymère carboxyvinylique | 0,5 |

### Phase C :

| | |
|---|---|
| - Triéthanolamine (neutralisant) | 0,5 |
| - Eau | 2,0 |

### Phase D :

| | |
|---|---|
| - Conservateur (méthylparabène) | 0,2 |
| - Glycérine | 5,0 |
| - Eau qsp | 100 |

### Mode opératoire :

On fait fondre les constituants de la phase A à 85° C, puis on refroidit la phase A à 70° C et on y introduit les phases B puis C et D sous agitation. On refroidit jusqu'à la température ambiante. On obtient une crème de jour hydratante qui agit contre le vieillissement naturel de la peau.

### TESTS SUR PEAU RECONSTRUITE (Système MATTEK)

La demanderesse a étudié la cytotoxicité (test au bromure de 3-(4,5-diMéthylThiazole-2-yl)-2,5-diphénylTétrazolium) d'émulsions conformes à l'exemple 2, dosées à différents pourcentages d'acide n-octanoyl-5-salicylique respectivement 0,25% ; 0,5% ; 1% et ceci en présence de concentrations croissantes de N-oléoyldihydro-sphingosine respectivement 0% ; 0,25% ; 0,50% ; 1% et 2%, sur un épiderme reconstruit issu de l'ensemencement de kératinocytes humains sur filtre Millipore recouvert de collagène.

100 mg d'émulsion sont incubés pendant 3 heures sur l'épiderme reconstruit (avec chaque mesure réalisée en double), puis la mesure de la viabilité cellulaire est réalisée immédiatement après rinçage de l'émulsion au "Phosphate Buffer Saline" (PBS).

L'expérimentation a été réalisée 3 fois sur 3 lots différents La figure 1 présente les résultats individuels de chaque lot et la figure 2 représente la moyenne des 3 lots. Les résultats obtenus sur les 3 lots concordent :
- après 3 heures d'incubation, les émulsions dosées à 0,25% d'acide n-octanoyl-5-salicylique ne présentent pas de cytotoxicité ;
- en présence de 0,5% d'acide n-octanoyl-5-salicylique, il apparaît un effet dose dépendant de la N-oléoyl-dihydrosphingosine sur la viabilité cellulaire. Cet effet permet une protection importante de viabilité cellulaire qui, en l'absence de la N-oléoyl-dihydrosphingosine est de 50% et en présence de 2% de la N-oléoyldihydrosphingosine atteint son taux maximum (100% de viabilité cellulaire) ;
- la concentration de 1% d'acide n-octanoyl-5-salicylique est , après 3 heures d'incubation, très cytotoxique ; il ne reste que 20% de cellules viables. Cette cytotoxicité se trouve néamoins diminuée par l'incorporation de la N-oléoyl-dihydrosphingosine au sein de la composition. En effet, la cytotoxicité moyenne obtenue avec 2% de la N-oléoyl-dihydrosphingosine est alors très proche de 50%.

## Revendications

1. Utilisation, dans une composition cosmétique ou dermatologique, contenant au moins un actif antivieillissement ayant un effet irritant et un milieu cosmétiquement ou dermatologiquement acceptable, d'un céramide de formule (I) suivante comme agent apaisant de l'effet irritant : 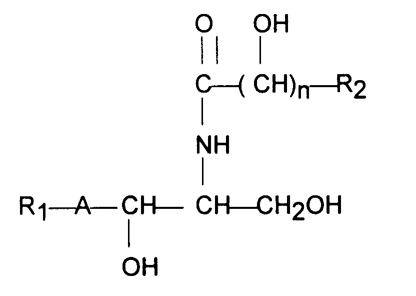 où A représente -CH₂-, 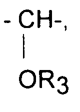 ou -CH=CH-;
R₁ représente une chaîne alkyle linéaire ou ramifiée, saturée ou insaturée, en C₁₀ à C₂₆ ;
R₂ représente une chaîne alkyle linéaire ou ramifiée, saturée ou insaturée, en C₁₂ à C₃₂ ;
R₃ représente H ou -CO-CHOH-R₂ ;
n représente 0 ou 1.

2. Utilisation selon la revendication précédente, caractérisée en ce que l'agent apaisant est choisi parmi la N-oléoyl-dihydrosphingosine, la N-stéaroyl phytosphingosine, la N-α-hydroxybéhénoyl-dihydrosphingosine et la N-α - hydroxypalmitoyl-dihydrosphingosine.

3. Utilisation selon la revendication 1 ou 2, caractérisée en ce que l'actif antivieillissement est choisi parmi les α-hydroxy-acides, β-hydroxy-acides α-céto-acides, β-céto-acides, rétinoïdes, anthralines, anthranoïdes, peroxydes, le minoxidil, la capsaïcine, les sels de lithium et de zinc, les antimétabolites, les vitamines.

4. Utilisation selon l'une quelconque des revendications précédentes, caractérisée en ce que l'actif antivieillissement est choisi parmi les α-hydroxy-acides, l'acide n-octanoyl-5-salicylique ou l'acide salicylique.

5. Utilisation selon l'une quelconque des revendications précédentes, caractérisée en ce que l'agent apaisant est utilisé dans un rapport en poids, agent apaisant/actif antivieillissement allant de 0,0001 à 100000.

6. Utilisation selon l'une quelconque des revendications précédentes, caractérisée en ce que la composition est sous forme d'une lotion, d'un gel aqueux, d'un sérum, d'une émulsion ou d'une dispersion de vésicules lipidiques.

7. Utilisation selon l'une quelconque des revendications précédentes, caractérisée en ce qu'elle contient des actifs lipophiles ou hydrophiles différents des actifs antivieillissement.

8. Utilisation de la composition définie dans l'une quelconque des revendications 1 à 7, pour traiter les rides et les ridules de la peau.
